# FASCICULE DE BREVET EUROPEEN

(11) **EP 3 556 422 B1**
(45) Date de publication et mention de la délivrance du brevet: **07.06.2023**
(21) Numéro de dépôt: 19170378.4
(22) Date de dépôt: 19.04.2019
(51) Int. Cl.: A61M 25/02, A61F 13/00

(54) **SET DE PANSEMENT PROTECTEUR DE CATHETER**
SCHUTZPFLASTERSET FÜR KATHETER
PROTECTIVE DRESSING SET FOR CATHETER

(30) Priorité: 19.04.2018 FR 1853470
(43) Date de publication de la demande: 23.10.2019
(73) Titulaire: LKN Médical, 42600 Montbrison (FR)
(72) Inventeur: PHILIPPON, Didier, 42600 MONTBRISON (FR)
(74) Mandataire: Cabinet Laurent & Charras

(56) Documents cités:
- WO-A-90/01351
- WO-A1-2015/166157
- WO-A2-2012/161897
- WO-A2-2014/039891
- US-A- 4 669 458

## Description

La présente invention concerne le domaine technique des dispositifs de maintien ou de fixation et de protection des cathéters de dialyse péritonéale ou d'hémodialyse (protection par pansements imperméables et respirants).

Le cathéter de dialyse péritonéale se présente sous forme d'un tube en silicone transparent qui sort du bas ventre des patients, permettant le remplissage ou la vidange du péritoine des patients avec une solution glucosée, afin de permettre les échanges entre le sang et cette solution. Cela permet d'épurer le sang de ses toxines urémiques, ainsi que de l'eau en excès.

Le cathéter d'hémodialyse se présente sous forme d'un ou deux tubes en silicone ou polyuréthane, sortant du thorax du patient au niveau de son pectoral, souvent après un trajet sous cutané (appelé tunellisation). Ce ou ces mêmes tubes entrent dans la veine jugulaire du patient et leurs extrémités descendent idéalement en limite de veine cave et d'oreillette. Ce système assure un accès au sang du patient, afin de procéder à son épuration lors des séances de dialyse.

### Art antérieur

Actuellement, de nombreux centres de dialyse réalisent leur propre système de pansement protecteur des cathéters de dialyse péritonéale ou d'hémodialyse, sans faire appel à un produit fini, complet et proposé dans un set de soin stérile. Par conséquent, il existe autant de protocoles que de centres de dialyse.

Le problème principal de la dialyse est la chronicité des patients, car ils sont traités à vie, sauf transplantation. Egalement, les habitudes du personnel soignant sont difficiles à changer.

Les patients en dialyse péritonéale (DP) ou en hémodialyse (HD) ont la plupart du temps des pansements collés à la peau 24h/24, ce qui va très souvent entraîner des réactions cutanées et allergies aux colles des adhésifs utilisés. Les dispositifs proposés actuellement ne permettent pas de résoudre toutes les allergies, tout en conservant une puissance de collage suffisante. En particulier, les enduits silicone permettent de résoudre les allergies, mais ne tiennent pas suffisamment collés à la peau.

Pour ces patients en DP ou HD, le point d'émergence de leur cathéter à travers la peau doit respirer afin d'éviter la macération, qui pourrait engendrer un risque infectieux.

WO2015/166157 décrit un set de pansement protecteur pour cathéter, comprenant une pièce en non tissé ou en mousse prévue pour être disposée au point d'émergence du cathéter, un fourreau prévu pour recevoir le tube du cathéter, et un pansement prévu pour recouvrir la pièce et l'ouverture supérieure du fourreau.

Ce set comprenant trois éléments distincts est relativement coûteux à fabriquer et complexe à mettre en oeuvre, nécessitant plusieurs manipulations par le patient ou le personnel médical.

WO2012161897A2 décrit (figure 3B) un autre set de pansement protecteur pour cathéter, correspondant au préambule des revendications 1 et 10. Le set est constitué d'un pansement et d'une pochette de maintien d'un connecteur externe du cathéter. Ce document ne précise pas le matériau de la pochette.

### Problèmes techniques posés

Ainsi, il existe un réel besoin de proposer un set de pansement permettant de protéger le point d'émergence du cathéter, et maintenir le connecteur externe du cathéter à proximité du pansement.

Dans le même temps, il est important de réduire le risque d'allergies, soit en réduisant les zones de collage, soit en faisant appel à des composants techniques répondant à cette problématique.

Actuellement, de nombreux patients sont forcés de se débrouiller avec leurs propres astuces (ceinture, pochette en tissu, gant de toilette...). Dans certains cas, en l'absence de solution adéquate ou faute de moyens financiers, les patients n'utilisent aucun pansement, ce qui est susceptible d'entrainer des risques importants pour leur santé.

### Résumé de l'invention

Le but de la présente invention est de proposer un set de pansement amélioré protecteur d'un cathéter de dialyse péritonéale ou d'hémodialyse, remédiant aux problèmes ci-dessus.

A cet effet, l'invention a pour objet un set de pansement protecteur pour cathéter de dialyse péritonéale ou d'hémodialyse, le set étant constitué:
- d'un pansement imperméable et respirant, conçu pour protéger un point d'émergence du cathéter, et comportant une fenêtre de visualisation du point d'émergence ; et
- d'un dispositif de maintien d'un connecteur externe du cathéter à proximité du pansement, le dispositif de maintien comprenant une pochette pourvue d'une ouverture sur au moins un côté ;
caractérisé en ce que la pochette est réalisée sous la forme d'un film en polyéthylène, viscose et polyester

Ainsi, l'invention permet de maintenir le cathéter de manière simple et pratique sur l'abdomen ou le thorax du patient, sans le gêner. L'invention est peu coûteuse à fabriquer et simple à utiliser.

L'invention évite que le cathéter ne se déplace dans les sous-vêtements ou vêtements du patient, et réduit donc le risque infectieux (péritonite ou endocardite). Le cathéter reste facilement accessible au patient, pour réaliser ses échanges (vider et remplir son ventre de solution glucosée) plusieurs fois par jour dans le cas DP.

La fenêtre de visualisation permet une surveillance médicale du point d'émergence du cathéter sortant de la peau du patient, ce qui permettra de traiter immédiatement tout début d'inflammation ou d'infection. De préférence, le dispositif de maintien permet d'occulter la fenêtre dans le cas DP, ou une compresse peut remplir ce rôle dans le cas HD, et ainsi empêcher le patient de visualiser ce point d'émergence, ce qui pourrait le gêner pour son image corporelle.

Le dispositif de maintien peut être fixé directement au pansement protégeant le point d'émergence, ce qui limite la zone de collage sur la peau. En alternative, le dispositif de maintien (de type pochette) peut être fixé directement à la peau du patient, puis être en partie recouvert par le pansement fenêtré.

Le pansement peut être changé de façon quotidienne ou tous les deux jours. Il doit donc être facile à réaliser, en respectant l'ensemble des règles d'aseptie afin de garantir une hygiène maximale. Le pansement est imperméable, pour permettre au patient de prendre une douche sans exposer le point d'émergence du cathéter à l'eau, ce qui pourrait lui faire courir un risque infectieux.

Le set de pansement selon l'invention peut être proposé à un coût raisonnable, dans un contexte où le remboursement des séances de dialyse ne cesse de baisser depuis plusieurs années.

Selon d'autres caractéristiques avantageuses de l'invention, prises isolément ou en combinaison :
- Le set est constitué d'un pansement et d'un dispositif de maintien.
- Le pansement est de préférence fenêtré.
- Le pansement et le dispositif de maintien sont deux éléments distincts.
- Le pansement et le dispositif de maintien sont solidarisés de manière amovible et non permanente.
- La pochette comprend une unique ouverture.
- La pochette est munie d'un système de fixation au pansement ou à la peau du patient
- Le système de fixation comprend au moins une bande adhésive équipant la pochette et prévue pour venir se fixer sur le pansement ou sur la peau.
- La bande adhésive comporte un papier protecteur, dépassant sur au moins un côté de la pochette pour faciliter sa saisie.
- Le papier protecteur est constitué de papier siliconé.
- La bande adhésive est double-face.
- La bande adhésive comporte une colle acrylique sur ses deux faces.
- Le système de fixation comprend au moins une bande à boucles ou crochets équipant la pochette, et prévue pour venir se fixer sur une bande à crochets ou boucles complémentaire équipant le pansement.
- Le système de fixation comprend deux bandes, de préférence parallèles, équipant la pochette.
- La pochette est opaque.
- La pochette mesure entre 3 et 15 cm de large par 7 à 20 cm de longueur, de préférence 7 cm de large sur 16 cm de longueur.
- La pochette comporte deux parois en vis-à-vis et une liaison latérale reliant ces deux parois.
- La liaison latérale est une soudure ou une couture.
- La liaison est respirante.
- La liaison est réalisée manuellement.
- La pochette est respirante et imperméable.
- La pochette disposée sur le pansement occulte la fenêtre de visualisation du point d'émergence (cas DP).
- Le pansement fenêtré recouvrant au moins partiellement la pochette occulte l'ouverture recevant le tube et le connecteur externe du cathéter (cas HD).
- Le pansement fenêtré comporte au moins une encoche latérale pour le passage et le maintien imperméable du tube du cathéter (cas DP).
- L'encoche latérale comprend une portion arrondie pour le passage du tube et une portion amincie prévue pour être resserrée autour du tube (cas DP).
- Le pansement fenêtré comprend : une couche externe transparente ; et une couche interne en forme de cadre délimitant la fenêtre de visualisation.
- La couche interne comporte une face interne adhésive, et le pansement comprend un dispositif couvrant la face interne adhésive et amovible pour appliquer la face interne adhésive contre la peau du patient.
- La couche interne comporte une face interne non-adhésive, apte à recevoir une application de matière adhésive.
- La couche externe est en polyuréthane.
- La couche interne est en matériau non-tissé
- La couche interne est en polyuréthane.
- Lorsque les couches interne et externe sont réalisées en polyuréthane, la couche interne est perforée.
- Lorsque les couches interne et externe sont réalisées en polyuréthane, la couche interne est perforée sur 20 à 40%, de préférence environ 30%, de la surface bordant la fenêtre centrale et où le polyuréthane est double.
- Les perforations ont un diamètre de 2,8 mm chacune.
- Le pansement fenêtré est réalisé en polyuréthane, émulsion acrylique, papier siliconé, viscose et/ou polyamide.
- Le pansement fenêtré a une couche de polyuréthane ayant une épaisseur de 25 µm.
- Le pansement fenêtré peut être enduit d'un adhésif du type gel silicone. De préférence, le gel silicone a une densité de 150 g/m².
- Le pansement fenêtré peut aussi être enduit d'un adhésif du type colle acrylique.
- Afin d'éviter une dégradation prématurée des propriétés adhésives du gel silicone ou de la colle acrylique, des bandes anti-adhésives de papier siliconé sont disposées au-dessus de la couche d'adhésif, par exemple en polyéthylène ou PET transparent.
- Le pansement fenêtré mesure entre 8x8 cm et 16x16 cm, de préférence 12 cm de largeur sur 14 cm de longueur.
- La quantité d'eau évacuée pendant 24h par le pansement est strictement supérieure à 1500 g/m².
- Le pansement est stérilisé selon les normes ISO 11135 et ISO 10993-7.
- Le pansement est dépourvu de latex, de phtalates, de bisphénols, de dérivés d'origine animale. Il est donc biocompatible.

Selon un autre mode de réalisation défini par la revendication 10, le set de pansement est caractérisé en ce que le dispositif de maintien est constitué d'une ou plusieurs bandelettes, chacune pourvue de deux extrémités adhésives et d'une partie centrale non-adhésive (cas DP).

Selon des caractéristiques particulières de ce mode de réalisation :
- Le dispositif de maintien comprend plusieurs bandelettes (cas DP).
- La bandelette comprend une unique partie non-adhésive, et non plusieurs parties non-adhésives réparties sur sa longueur (cas DP).

### Description des figures

L'invention sera mieux comprise à la lecture de la description qui va suivre, donnée uniquement à titre d'exemple non limitatif et faite en référence aux dessins annexés sur lesquels :
- les figures 1 et 2 sont des vues de face, respectivement d'un pansement et d'un dispositif de maintien sous forme de pochette, formant un set de pansement conforme à un premier mode de réalisation de l'invention ;
- la figure 3 est une vue de face du ventre d'un patient traité par dialyse péritonéale, avec un point d'émergence d'un cathéter ;
- la figure 4 est une vue analogue à la figure 3, montrant le ventre du patient équipé du set de pansement des figures 1 et 2 ;
- la figure 5 est une vue à plus grande échelle du détail V à la figure 4, montrant le passage et le maintien imperméable du tube du cathéter dans une encoche latérale du pansement ;
- la figure 6 est une vue analogue à la figure 4, montrant le cathéter maintenu dans la pochette ;
- la figure 7 est une vue en perspective du ventre et du set de pansement, lorsque la pochette est soulevée pour voir le point d'émergence du cathéter à travers la fenêtre du pansement ;
- la figure 8 est une vue de face du ventre d'un patient ayant subi une dialyse péritonéale, équipé d'un set de pansement conforme à un deuxième mode de réalisation de l'invention ;
- la figure 9 est une vue analogue à la figure 8, montrant une variante du deuxième mode de réalisation ;
- la figure 10 est une vue analogue à la figure 1, montrant un pansement conforme à un troisième mode de réalisation de l'invention, dépourvu d'encoche pour le passage de la tubulure ;
- la figure 11 est une vue partielle de côté, et à plus grande échelle, centrée sur le détail XI, du pansement de la figure 10 ;
- la figure 12 est une vue analogue à la figure 11, montrant un pansement conforme à un quatrième mode de réalisation de l'invention ;
- les figures 13 et 14 sont des vues analogues aux figures 3 et 4, montrant un set de pansement conforme à un cinquième mode de réalisation de l'invention.

### Description détaillée de l'invention

La description ci-après fait référence aux manipulations du patient dans le cadre de la DP, étant entendu que les mêmes manipulations pourraient être effectuées par le personnel soignant ou par un proche, et nécessairement par un personnel soignant lors de l'HD.

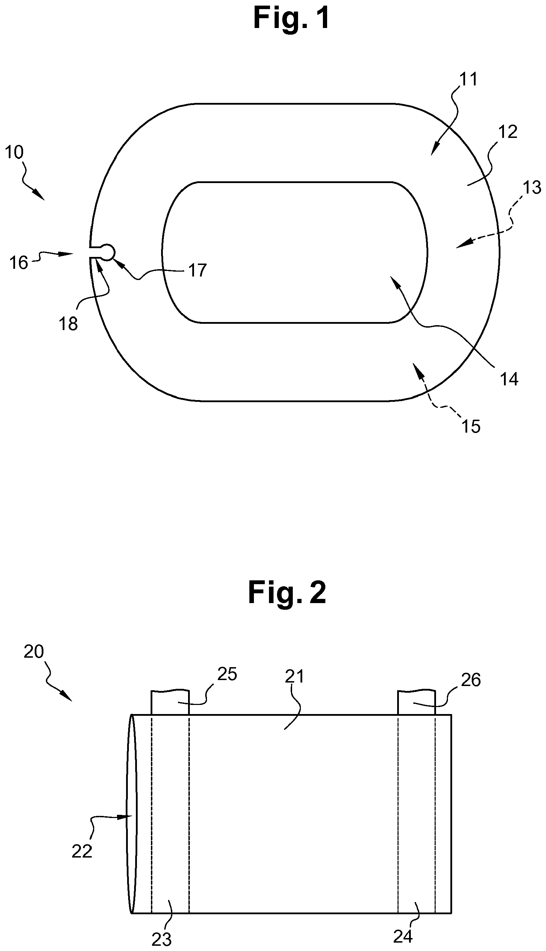

Les figures 1 et 2 montrent un set de pansement protecteur de cathéter pour DP, comprenant un pansement (10) et un dispositif de maintien (20) se présentant sous forme de pochette (21).

Ce set est constitué de seulement deux éléments distincts, à savoir le pansement (10) et le dispositif de maintien (20). Cela facilite grandement ses manipulations. Le set est pratique à utiliser et présente un coût réduit, en comparaison avec des sets plus complexes.

Le pansement (10) est conçu pour protéger un point d'émergence du cathéter DP sortant du ventre d'un patient. Le pansement fenêtré (10) est imperméable et respirant.

Le pansement (10) comprend une couche externe (11) imperméable et transparente, une couche interne (12) opaque (ou transparente) en forme de cadre et un papier protecteur (13) amovible recouvrant une face interne (15) de la couche interne (12), à l'opposé de la couche externe (11).

La couche (12) délimite une fenêtre centrale (14), qui est recouverte par la couche (11) transparente non adhésive et permet de voir à travers le pansement (10). Les dimensions de la fenêtre (14) sont par exemple de l'ordre de 8cm x 4cm, mais peuvent varier en fonction du patient. La fenêtre (14) est dépourvue de matière adhésive. La fenêtre (14) transparente permet une surveillance médicale du point d'émergence du cathéter qui sort de la peau du patient. La pochette (21) permet d'occulter la fenêtre (14), et ainsi empêcher le patient de visualiser ce point d'émergence, pour une discrétion, une sécurité et un confort accrus.

La face interne (15) de la couche (12) est adhésive, pour fixer le pansement (10) contre la peau du patient. Initialement, la face (15) est recouverte par le papier protecteur (13), qui est amovible pour découvrir la face interne (15) et coller le pansement (10). La zone de collage de la face (15) est limitée à la forme du cadre de la couche (12), autour de la fenêtre (14). Ainsi, la zone de collage est réduite au maximum, ce qui permet de limiter l'utilisation de colle qui pourrait provoquer une réaction allergique désagréable ou nocive chez le patient. La colle utilisée est la plus hypoallergénique possible et/ou fait appel à une technique d'enduction permettant de réduire sa nocivité. L'utilisation d'enduits silicones ou PSA silicones est envisageable, par exemple sous forme de gel.

De préférence, la couche externe (11) est en polyuréthane (transparent), tandis que la couche interne (12) est en matériau non-tissé (opaque) ou en polyuréthane (transparent). En alternative, les couches (11, 12) peuvent être en d'autres matériaux adaptés à l'application visée. Le pansement (10) doit être respirant pour ne pas entraîner de macération contre la peau du patient. De préférence, au moins l'une des couches (11, 12) est perforée (ou en matière poreuse très respirante), pour améliorer la respirabilité et la flexibilité du pansement (10).

Le pansement (10) dans le cas de la DP comporte une encoche (16) latérale pour le passage et le maintien étanche du tube du cathéter de DP. L'encoche (16) est formée à travers les deux couches (11, 12). L'encoche (16) peut être en forme de « trou de serrure », avec une portion arrondie (17) pour le passage du tube et une portion amincie (18) prévue pour être resserrée autour du tube.

Le dispositif de maintien (20) est conçu pour maintenir le cathéter (3) à proximité du pansement (10), de préférence contre le pansement (10).

Le dispositif (20) comprend une pochette (21) constituée de deux parties délimitant entre elles un espace de réception du cathéter. De préférence, ces deux parties sont imperméables. Encore de préférence, la pochette (21) est opaque et de couleur blanche. Cela permet de masquer le tube (4) et le connecteur (5) du cathéter (3) logé dans la pochette (21).

La pochette (21) est pourvue d'une ouverture (22) sur au moins un côté, de préférence une unique ouverture (22) sur un côté, ou bien deux ouvertures (22) sur des côtés opposés.

Les dimensions de la pochette (21) sont de préférence comprises entre 7cm x 3cm et 15cm x 20cm, encore de préférence de l'ordre de 12cm x 7,5cm ou 16cm x 7cm. De telles dimensions permettent à la pochette (21) d'occulter la fenêtre (14), quel que soit le positionnement du pansement (10).

Une face de la pochette (21) est munie de deux bandes adhésives (23, 24) doubles faces parallèles, pour sa fixation au pansement (10). Chaque bande (23, 24) comporte une face solidaire de la pochette (21) et une face collante opposée à la pochette (21). La face collante est munie d'un papier protecteur (25, 26) amovible. Une fois le papier protecteur (25, 26) retiré, la face collante peut être appliquée sur le pansement (10) pour y fixer la pochette (21).

Avantageusement, les papiers protecteurs (25, 26) des bandes adhésives (23, 24) peuvent dépasser sur un côté de la pochette (21) différent de l'ouverture (22), pour faciliter leur saisie par le patient.

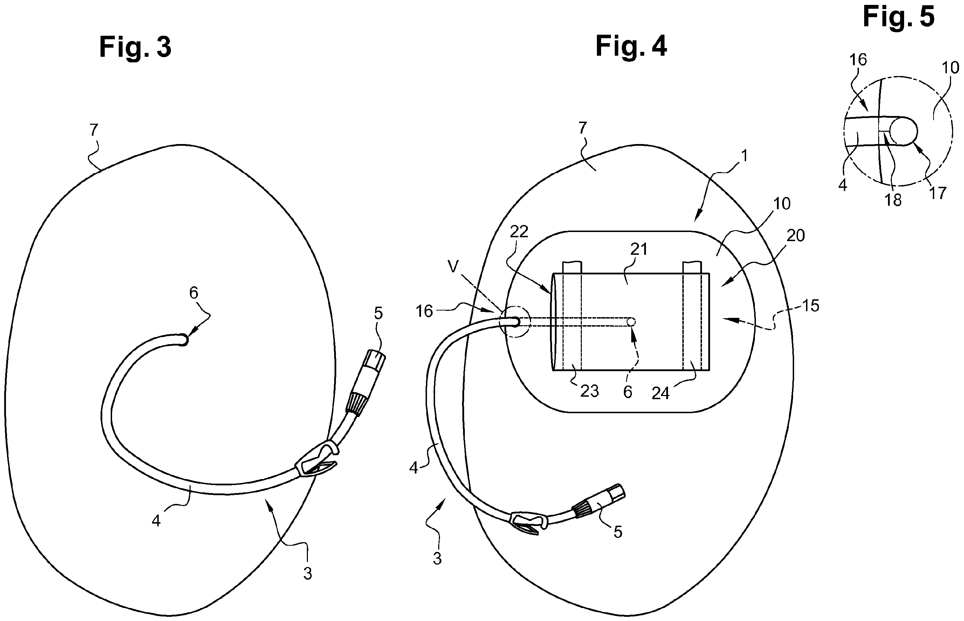

La figure 3 montre le cathéter de DP (3) sortant du ventre (7) du patient à un point d'émergence (6). Le cathéter (3) comprend un tube (4) sortant du ventre (7) et un connecteur externe (5) pour connecter le cathéter à une poche de solution glucosée posée ou suspendue sur un appareil de dialyse. Le connecteur (5) est conçu pour recevoir un autre tube, afin de relier le tube (4) de cathéter (3) à la poche.

Les figures 4 à 6 montrent le set de pansement (1) équipant le patient. Le pansement (10) est collé sur le ventre (7) par sa face interne (15) adhésive. La fenêtre (14) est centrée sur le point d'émergence (6), non visibles sous la pochette (21).

Le tube (4) s'étend sur le côté du pansement (10) et ressort par l'encoche (16), plus précisément par la portion arrondie (17), tandis que la portion amincie (18) est resserrée autour du tube (4). Cela permet de garantir l'imperméabilité du pansement (10) contre la peau du ventre (7) à la sortie du tube (4).

La pochette (21) est collée sur la couche externe (11) du pansement (10) via les bandes adhésives (23, 24). La pochette (21) recouvre alors la fenêtre (14), ce qui permet de masquer le point d'émergence (6) au patient.

Le connecteur (5) et le tube (4) du cathéter (3) peuvent être facilement glissés dans la pochette (21) par le patient, de manière à les maintenir contre le pansement (10) sans provoquer de gêne. Le poids du cathéter (3) est réparti sur tout le volume interne de la pochette (21). Lorsqu'une nouvelle opération de dialyse doit être effectuée, le patient peut facilement sortir le connecteur (5) et le tube (4) hors de la pochette (21).

Ainsi, le cathéter (3) peut être maintenu de manière simple et pratique sur le ventre du patient, sans engendrer de surface de collage supplémentaire contre la peau.

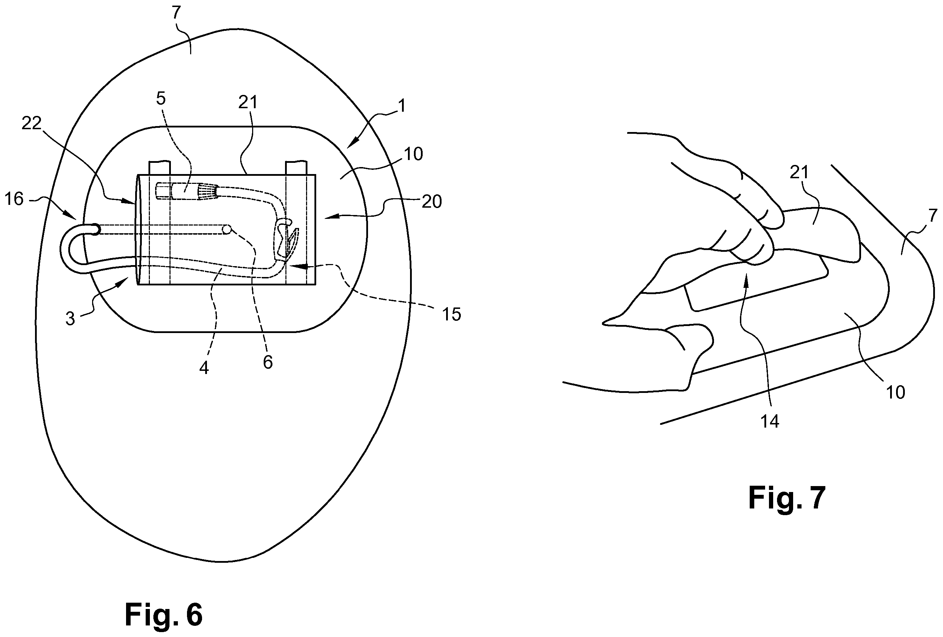

La figure 7 montre une personne soulevant légèrement la pochette (21), ce qui lui permet de voir à travers la fenêtre (14). Cette personne peut être le patient ou un membre du personnel soignant. Cela permet de vérifier qu'aucune infection ne s'est développée au niveau du point d'émergence (6) du cathéter (3).

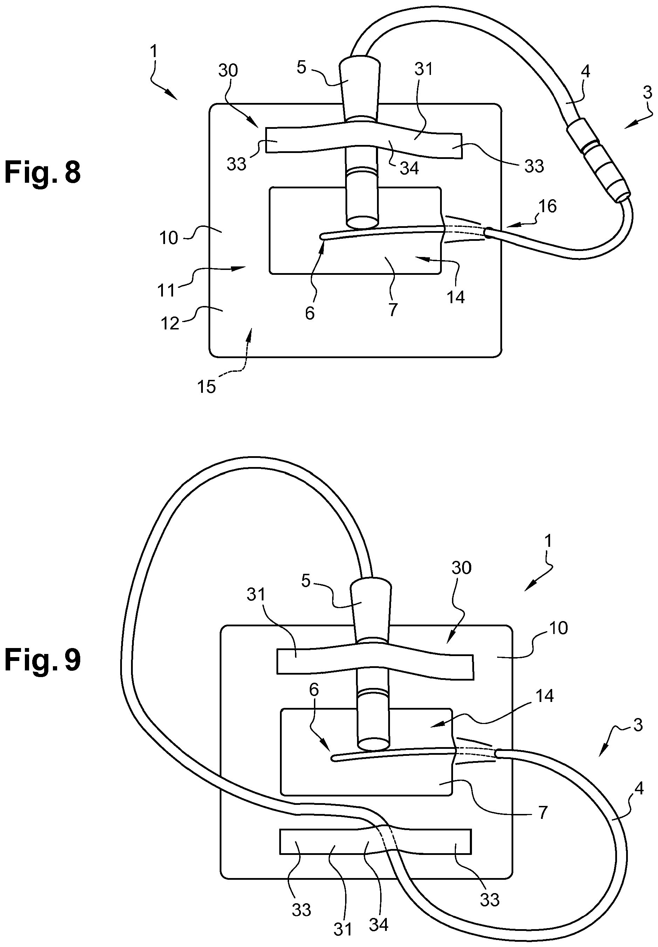

La figure 8 montre un deuxième mode de réalisation d'un set de pansement (1) conforme à l'invention. Certains éléments constitutifs du set (1) sont comparables à ceux du premier mode de réalisation décrit plus haut et, dans un but de simplification, portent les mêmes références numériques.

Le set (1) est constitué d' un pansement (10) et d'un dispositif de maintien (30), sous forme de bandelette (31) pourvue de deux extrémités (33) adhésives et d'une partie centrale (34) non-adhésive.

Grâce à la bandelette (31), le connecteur (5) et le tube (4) du cathéter (3) peuvent être facilement fixés au pansement (10) par le patient, de manière à les maintenir contre le pansement (10) sans provoquer de gêne. Lorsqu'une nouvelle opération de dialyse doit être effectuée, le patient peut facilement détacher le connecteur (5).

Ainsi, le cathéter (3) peut être maintenu de manière simple et pratique sur le ventre du patient, sans engendrer de surface de collage supplémentaire contre la peau.

La figure 9 montre une variante de la figure 8, dans laquelle le dispositif de maintien (30) comprend deux bandelettes (31). Une bandelette (31) est utilisée pour tenir le tube (4), tandis que la seconde bandelette (31) est utilisée pour tenir le connecteur (5). Le set de pansement (1) peut comprendre plusieurs pansements (10) et bandelettes (31), de sorte que le patient peut utiliser le nombre requis de bandelettes (31) avec chaque pansement (10), en fonction de la longueur du tube (4).

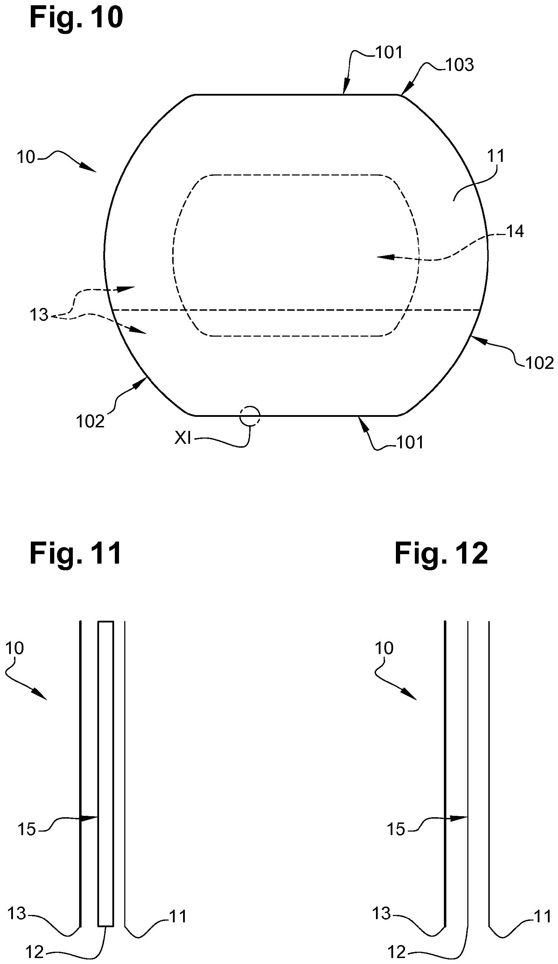

Les figures 10 et 11 montrent une variante du pansement (10) des figures 1 à 6. La figure 10 est une vue de face, tandis que la figure 11 est une vue en coupe du pansement (10). Le pansement (10) est constitué d'une couche externe (11) en polyuréthane collée à une couche (12) en non-tissé. De préférence, la couche (11) en polyuréthane mesure 25 µm d'épaisseur.

La face interne (15) de la couche (12) est adhésive, pour fixer le pansement (10) contre la peau du patient ou sur la pochette (21). Initialement, la face (15) est recouverte par le papier protecteur (13), qui est amovible pour découvrir la face interne (15) et coller le pansement (10). La zone de collage de la face (15) est limitée à la forme du cadre de la couche (12), autour de la fenêtre (14).

Les dimensions du pansement (10) sont 14cm x 12cm, tandis que les dimensions de la fenêtre (14) sont 9cm x 6cm. Le pansement (10) a une géométrie particulière, assurant sa résistance au décollement. Le pansement (10) a deux côtés droits (101) et deux côtés arrondis (102), deux à deux opposés, et séparés par quatre coins (103). Les côtés arrondis (102) ont un rayon de courbure de 7,25cm, tandis que les coins (103) ont un rayon de courbure de 0,1cm.

La figure 12 est une variante de la figure 11, où le pansement (10) comprend deux couches (11, 12) en polyuréthane. La couche interne (12) est perforée pour assurer la respirabilité du pansement (10). De préférence, la couche interne (12) est perforée sur 20 à 40%, par exemple environ 30%, de la surface bordant la fenêtre centrale (14). Encore de préférence, les perforations ont un diamètre de 2,8 mm chacune.

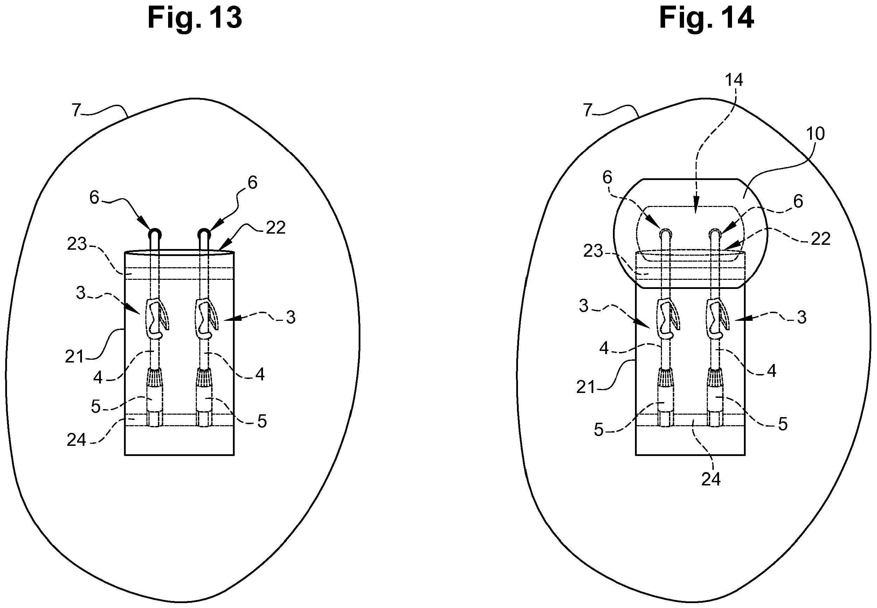

Les figures 13 et 14 montrent une variante d'un set de pansement (1) conforme à l'invention, dans le cas d'une hémodialyse (HD).

Le cathéter d'hémodialyse (3) comprend un ou deux tubes (4) sortant du pectoral (7) du patient à un ou deux point(s) d'émergence(s) (6). Le cathéter (3) comprend également un ou deux connecteur(s) externe(s) (5) munis de clamps. Le(s) connecteur(s) externe(s) (5) seront reliés durant la séance d'hémodialyse à un générateur de dialyse et un filtre (hémodialyseur) afin d'extraire le sang du patient et en assurer l'épuration, durant une séance de 4h en moyenne.

Le set de pansement (1) comprend une pochette (21) fixée en partie haute sur la peau du patient, et un pansement (10) recouvrant partiellement la pochette (21). En particulier, le pansement (10) recouvre l'ouverture (22) et de préférence au moins 4cm en partie supérieure de la pochette (21). Le pansement (10) assure l'étanchéité avec la peau du patient sur les bords de la pochette (21).

La face arrière de la pochette (21) est équipée de bandelettes (23, 24) adhésives double face, respectivement en partie supérieure et en partie inférieure. La bandelette supérieure (23) permet de coller la pochette (21) sur la peau du patient. La bandelette inférieure (24) peut être utilisée pour ajuster la longueur de la pochette (21) à la longueur des tubes (4) et connecteurs (5), en repliant la pochette (21) sur elle-même.

Dans le cas HP, le pansement fenêtré (10) est dépourvu d'encoche latérale. La fenêtre de visualisation (14) du pansement (10) peut être occultée par une compresse, non représentée sur la figure 14 dans un but de simplification. Ainsi, la compresse empêche le patient de visualiser les points d'émergence (6), ce qui pourrait le gêner pour son image corporelle.

Par ailleurs, le set de pansement (1) peut être conformé différemment des figures 1 à 14 sans sortir du cadre de l'invention, définie par les revendications.

En variante non représentée, le pansement (10) peut être dépourvu de papier protecteur (13), tandis que la couche interne (12) comporte une face interne (15) non-adhésive, apte à recevoir une application de matière adhésive. Dans ce cas, le set de pansement (1) peut inclure un tube ou un pot de colle. Le patient applique lui-même la colle sur la couche (12) autour de la fenêtre (14), autrement dit sur la face interne (15) en forme de cadre.

Selon une autre variante non représentée, la pochette (21) et le pansement (10) sont munis d'un système à boucles et crochets, permettant de fixer la pochette (21) au pansement (10).

Selon une autre variante non représentée, la pochette (21) est dépourvue de système de fixation au pansement (10). Le patient peut appliquer lui-même de la colle sur la pochette (21) pour la fixer au pansement (10).

Selon une autre variante non représentée, le pansement (10) peut comporter plusieurs encoches latérales (16), notamment sur différents côtés, au lieu d'une unique encoche latérale (16) sur un seul côté.

En outre, les caractéristiques techniques des différents modes de réalisation et variantes mentionnés ci-dessus peuvent être, en totalité ou pour certaines d'entre elles, combinées entre elles.

Ainsi, le set de pansement (1) peut être adapté en termes de coût, d'ergonomie, de fonctionnalités et de performance.

## Revendications

1. Set (1) de pansement protecteur d'un cathéter (3) de dialyse péritonéale ou d'hémodialyse, le set (1) étant constitué :
- d'un pansement (10) imperméable et respirant, conçu pour protéger un point d'émergence (6) du cathéter (3), et comportant une fenêtre (14) de visualisation du point d'émergence (6) ; et
- d'un dispositif de maintien (20) d'un connecteur externe (5) du cathéter (3) à proximité du pansement (10), le dispositif de maintien (20) comprenant une pochette (21) pourvue d'une ouverture (22) sur au moins un côté ; **caractérisé en ce que** la pochette (21) est réalisée sous la forme d'un film en polyéthylène, viscose et polyester.

2. Set (1) selon la revendication 1, **caractérisé en ce que** la pochette (21) est munie d'un système de fixation au pansement (10) ou à la peau du patient.

3. Set (1) selon la revendication 2, **caractérisé en ce que** le système comprend au moins une bande adhésive (23 ; 23, 24).

4. Set (1) selon la revendication 3, **caractérisé en ce que** le système comprend deux bandes adhésives (23, 24) parallèles.

5. Set (1) selon la revendication 3 ou 4, **caractérisé en ce que** la ou chaque bande adhésive (23 ; 23, 24) comporte un papier protecteur (25 ; 25, 26) dépassant sur au moins un côté de la pochette (21) pour faciliter sa saisie par un patient, un personnel soignant ou un proche.

6. Set (1) selon l'une des revendications précédentes, **caractérisé en ce que** la pochette (21) est opaque.

7. Set (1) selon la revendication 6, **caractérisé en ce que** la pochette (21) disposée sur le pansement (10) occulte la fenêtre (14) de visualisation du point d'émergence (6).

8. Set (1) selon l'une des revendications 2 à 6, **caractérisé en ce que** le pansement (10) recouvrant au moins partiellement la pochette (21) occulte l'ouverture (22) recevant le tube (4) et le connecteur externe (5) du cathéter (3).

9. Set (1) selon l'une des revendications précédentes, **caractérisé en ce que** la pochette (21) mesure entre 3 et 15 cm de large par 7 à 20 cm de longueur, de préférence 7 cm de large sur 16 cm de longueur.

10. Set (1) de pansement protecteur d'un cathéter (3) de dialyse péritonéale ou d'hémodialyse, le set (1) étant constitué :
- d'un pansement (10) imperméable et respirant, conçu pour protéger un point d'émergence (6) du cathéter (3), et comportant une fenêtre (14) de visualisation du point d'émergence (6) ; et
- d'un dispositif de maintien (20) d'un connecteur externe (5) du cathéter (3) à proximité du pansement (10) ;
**caractérisé en ce que** le dispositif de maintien (30) est constitué d'une ou plusieurs bandelettes (31), chacune pourvue de deux extrémités (33) adhésives et d'une partie centrale (34) non-adhésive.

11. Set (1) selon l'une des revendications précédentes, **caractérisé en ce que** le pansement (10) comporte une encoche (16) latérale pour le passage et le maintien imperméable du tube (4) du cathéter (3).

12. Set (1) selon l'une des revendications précédentes, **caractérisé en ce que** le pansement (10) comprend :
a. une couche externe (11) transparente ; et
b. une couche interne (12) en forme de cadre délimitant la fenêtre (14) de visualisation.

13. Set (1) selon la revendication 12, **caractérisé en ce que** la couche externe (11) est en polyuréthane.

14. Set (1) selon l'une des revendications 12 ou 13, **caractérisé en ce que** la couche interne (12) est en polyuréthane.

15. Set (1) selon l'une des revendications 12 ou 13, **caractérisé en ce que** la couche interne (12) est en matériau non-tissé.

## Patentansprüche

1. Set (1) eines Schutzverbands für einen Katheter (3) der Peritonealdialyse oder Hämodialyse, wobei das Set (1) zusammengesetzt ist
- aus einem undurchlässigen und atmungsaktiven Verband (10), der ausgelegt ist, eine Austrittsstelle (6) des Katheters (3) zu schützen, und ein Sichtfenster (14) für die Austrittsstelle (6) umfasst; und
- aus einer Vorrichtung (20) zum Halten eines äußeren Verbindungsstücks (5) des Katheders (3) in der Nähe des Verbands (10), wobei die Haltevorrichtung (20) eine Tasche (21) umfasst, die auf zumindest einer Seite mit einer Öffnung (22) versehen ist, **dadurch gekennzeichnet, dass** die Tasche (21) in Form einer Folie aus Polyethylen, Viskose und Polyester ausgeführt ist.

2. Set (1) nach Anspruch 1, **dadurch gekennzeichnet, dass** die Tasche (21) mit einem System der Befestigung am Verband (10) oder an der Haut des Patienten ausgestattet ist.

3. Set (1) nach Anspruch 2, **dadurch gekennzeichnet, dass** das System zumindest einen Klebestreifen (23; 23, 24) umfasst.

4. Set (1) nach Anspruch 3, **dadurch gekennzeichnet, dass** das System zumindest zwei parallele Klebestreifen (23, 24) umfasst.

5. Set (1) nach Anspruch 3 oder 4, **dadurch gekennzeichnet, dass** der oder jeder Klebestreifen (23; 23, 24) ein Schutzpapier (25; 25, 26) umfasst, das an zumindest einer Seite über die Tasche (21) hinausragt, um das Ergreifen der Tasche durch einen Patienten, eine Pflegekraft oder einen Angehörigen zu erleichtern.

6. Set (1) nach einem der vorstehenden Ansprüche, **dadurch gekennzeichnet, dass** die Tasche (21) opak ist.

7. Set (1) nach Anspruch 6, **dadurch gekennzeichnet, dass** die am Verband (10) angeordnete Tasche (21) das Sichtfenster (14) der Austrittsstelle (6) verdeckt.

8. Set (1) nach einem der Ansprüche 2 bis 6, **dadurch gekennzeichnet, dass** der zumindest teilweise die Tasche (21) abdeckende Verband (10) die den Schlauch (4) und das äußere Verbindungsstück (5) des Katheters (3) aufnehmende Öffnung (22) verbirgt.

9. Set (1) nach einem der vorstehenden Ansprüche, **dadurch gekennzeichnet, dass** die Tasche (21) als Abmessungen eine Breite zwischen 3 und 15 cm und eine Länge von 7 bis 20 cm, bevorzugt eine Breite von 7 cm und eine Länge von 16 cm, aufweist.

10. Set (1) eines Schutzverbands für einen Katheter (3) der Peritonealdialyse oder Hämodialyse, wobei das Set (1) zusammengesetzt ist
- aus einem undurchlässigen und atmungsaktiven Verband (10), der ausgelegt ist, eine Austrittsstelle (6) des Katheters (3) zu schützen, und ein Sichtfenster (14) der Austrittsstelle (6) umfasst; und
- aus einer Vorrichtung zum Halten (20) eines äußeren Verbindungsstücks (5) des Katheders (3) in der Nähe des Verbands (10);
**dadurch gekennzeichnet, dass** die Haltevorrichtung (30) aus einem oder mehreren Streifen (31) besteht, wobei jeder Streifen mit zwei klebenden Enden (33) und einem nicht klebenden mittleren Abschnitt (34) ausgestattet ist.

11. Set (1) nach einem der vorstehenden Ansprüche, **dadurch gekennzeichnet, dass** der Verband (10) einen seitlichen Einschnitt (16) zum Durchführen und undurchlässigen Halten des Schlauchs (4) des Katheders (3) umfasst.

12. Set (1) nach einem der vorstehenden Ansprüche, **dadurch gekennzeichnet, dass** die Tasche (10) Folgendes umfasst:
a. eine äußere transparente Schicht (11); und
b. eine innere Schicht (12) in Form eines Rahmens, der das Sichtfenster (14) begrenzt.

13. Set (1) nach Anspruch 12, **dadurch gekennzeichnet, dass** die äußere Schicht (11) aus Polyurethan besteht.

14. Set (1) nach einem der Ansprüche 12 oder 13, **dadurch gekennzeichnet, dass** die innere Schicht (12) aus Polyurethan besteht.

15. Set (1) nach einem der Ansprüche 12 oder 13, **dadurch gekennzeichnet, dass** die innere Schicht (12) aus Vlies besteht.

## Claims

1. A protective dressing set (1) of a peritoneal dialysis or haemodialysis catheter (3), the set (1) being constituted by:
- an impermeable and breathable dressing (10) designed to protect an exit point (6) of the catheter (3), and comprising a window (14) for viewing the exit point (6); and
- a device (20) for holding an outer connector (5) of the catheter (3) close to the dressing (10), the holding device (20) comprising a pouch (21) provided with an opening (22) on at least one side;
**characterized in that** the pouch (21) is produced in the form of a film of polyethylene, viscose and polyester.

2. Set (1) as claimed in claim 1, **characterized in that** the pouch (21) is provided with a system for fastening to the dressing (10) or to the skin of the patient.

3. Set (1) as claimed in claim 2, **characterized in that** the system comprises at least one adhesive strip (23; 23, 24).

4. Set (1) as claimed in claim 3, **characterized in that** the system comprises two parallel adhesive strips (23, 24).

5. Set (1) as claimed in claim 3 or claim 4, **characterized in that** the or each adhesive strip (23; 23, 24) comprises a protective paper (25; 25, 26) protruding out on at least one side of the pouch (21) in order to facilitate grasping thereof by a patient, a caregiver or a relative.

6. Set (1) as claimed in one of the preceding claims, **characterized in that** the pouch (21) is opaque.

7. Set (1) as claimed in claim 6, **characterized in that** the pouch (21) disposed on the dressing (10) obscures the window (14) for viewing the exit point (6).

8. Set (1) as claimed in one of claims 2 to 6, **characterized in that** the dressing (10) which at least partially covers the pouch (21) obscures the opening (22) receiving the tube (4) and the outer connector (5) of the catheter (3).

9. Set (1) as claimed in one of the preceding claims, **characterized in that** the pouch (21) measures between 3 and 15 cm in width by 7 to 20 cm in length, preferably 7 cm in width by 16 cm in length.

10. A protective dressing set (1) of a peritoneal dialysis or haemodialysis catheter (3), the set (1) being constituted by:
- an impermeable and breathable dressing (10) designed to protect an exit point (6) of the catheter (3), and comprising a window (14) for viewing the exit point (6); and
- a device (20) for holding an outer connector (5) of the catheter (3) close to the dressing (10);
**characterized in that** the holding device (30) is constituted by one or more strips (31), each provided with two adhesive ends (33) and a non-adhesive central portion (34).

11. Set (1) as claimed in one of the preceding claims, **characterized in that** the dressing (10) comprises a lateral notch (16) for the passage of and for impermeably holding the tube (4) of the catheter (3).

12. Set (1) as claimed in one of the preceding claims, **characterized in that** the dressing (10) comprises:
a. a transparent outer layer (11); and
b. an inner layer (12) in the form of a frame defining the viewing window (14).

13. Set (1) as claimed in claim 12, **characterized in that** the outer layer (11) is produced from polyurethane.

14. Set (1) as claimed in one of claims 12 or 13, **characterized in that** the inner layer (12) is produced from polyurethane.

15. Set (1) as claimed in one of claims 12 or 13, **characterized in that** the inner layer (12) is produced from non-woven material.
